# FASCICULE DE BREVET EUROPEEN

(11) **EP 0 809 620 B1**
(45) Date de publication et mention de la délivrance du brevet: **07.04.1999**
(21) Numéro de dépôt: 96901400.0
(22) Date de dépôt: 16.01.1996
(51) Int. Cl.: C07C 17/23, C07C 17/269, C07C 19/08

(54) **PROCEDE DE COPRODUCTION DE DIFLUOROMETHANE ET DE 1,1,1,2-TETRAFLUOROETHANE**
VERFAHREN ZUR COPRODUKTION VON DIFLUORMETHAN UND 1,1,1,2-TETRAFLUORETHAN
METHOD FOR COPRODUCING DIFLUOROMETHANE AND 1,1,1,2-TETRAFLUOROETHANE

(30) Priorité: 17.02.1995 FR 9501859
(43) Date de publication de la demande: 03.12.1997
(73) Titulaire: ELF ATOCHEM S.A., 92800 Puteaux, Hauts-de-Seine (FR)
(72) Inventeur: SCHIRMANN, Jean-Pierre, F-75011 Paris (FR); HUB, Serge, F-69100 Villeurbanne (FR); LANTZ, André, F-69390 Vernaison (FR)
(74) Mandataire: Leboulenger, Jean
(86) Numéro de dépôt international: FR9600070
(87) Numéro de publication internationale: WO9625377

(56) Documents cités:
- EP-A- 0 508 660
- WO-A-91/09000
- WO-A-95/24369

## Description

La présente invention concerne la fabrication simultanée de difluorométhane (F32) et de 1,1,1,2-tétrafluoroéthane (F134a) par pyrolyse de chlorodifluorométhane en présence d'hydrogène, en opérant à une température supérieure à 500°C en l'absence de tout catalyseur ou surface métallique. Suivant les conditions opératoires choisies, ce procédé permet également de fabriquer de façon majoritaire soit du F134a, soit du F32.

Le 1,1,1,2-tétrafluoroéthane (F134a) est déjà fabriqué industriellement pour remplacer le difluorodichlorométhane (F12) dans la réfrigération domestique et la climatisation automobile en particulier. Deux voies sont actuellement utilisées commercialement. Elles mettent en oeuvre des procédés qui consistent :
- soit à fluorer le trichloréthylène en deux étapes 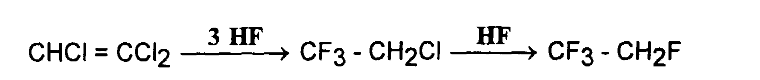
- soit à fluorer le perchloréthylène, puis à hydrogénolyser selon le cas du 1,1-dichloro-1,2,2,2-tétrafluoroéthane (F114a) ou du 1-chloro-1,2,2,2-tétrafluoroéthane (F124).

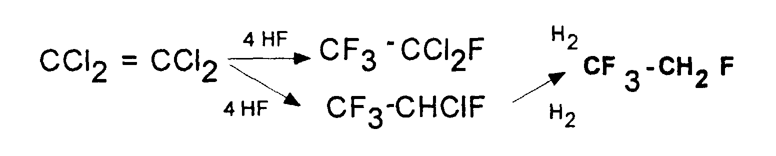

Ces procédés nécessitent des unités neuves particulièrement onéreuses par les investissements et la nécessité d'employer des catalyseurs très sophistiqués dont la durée de vie et les performances ne sont pas sans effet sur le coût du produit commercial.

Le difluorométhane CH₂F₂ (F32) est un composé potentiellement très intéressant, puisqu'en association avec le pentafluoroéthane (F125) il donne un mélange azéotropique qui est un excellent produit de substitution au chlorodifluorométhane (F22) dont l'interdiction est programmée entre 2005 et 2015 en raison de son effet potentiel sur l'affaiblissement de la couche d'ozone stratosphérique.

Le chlorodifluorométhane est utilisé à grande échelle comme fluide frigorifique pour la réfrigération commerciale et pour la climatisation des grands immeubles.

Les voies retenues pour fabriquer le F32 commercialement sont au nombre de quatre :
**1**. Fluoration du chlorure de méthylène CH₂Cl₂ par HF en phase liquide en présence d'un catalyseur à base d'antimoine.
**2**. Fluoration du chlorure de méthylène CH₂Cl₂ par HF en phase gaz sur un catalyseur à base de chrome et à haute température.
Ces deux procédés ne sont pas satisfaisants parce qu'ils impliquent la coproduction d'importantes quantités de chlorofluorométhane (F31) hautement toxique (CL 50 = 2 ppm).
**3**. Hydrogénolyse du chlorodifluorométhane (F22) en présence d'un catalyseur à base de métal précieux à des températures comprises entre 200 et 300°C, ou en présence de métaux tels que l'aluminium, le molybdène, le titane, le nickel, le fer ou le cobalt à une température comprise entre 300 et 700°C comme dans la demande de brevet WO 91/05752.
   L'hydrogénolyse n'est pas du tout sélective et conduit à une formation importante de méthane à côté de quantités moindres de F 31.
**4**. Un nouveau procédé de fabrication de F32 proposé plus récemment, éliminant la coproduction de F31 mais très lourd et peu sélectif, est basé sur la fluoration par HF du formaldéhyde telle que décrite dans la demande de brevet EP 518 506.

   2 CH₂O + 2 HF → H₂O + FCH₂ - O - CH₂F → CH₂F₂ + CH₂O

Ce procédé requiert deux étapes et co-produit de l'eau en présence d'acide fluorhydrique, induisant des risques de corrosion élevés.

Le chlorodifluorométhane (F22) est fabriqué actuellement à grande échelle pour la réfrigération commerciale mais aussi pour servir de matière première pour la production de PTFE. Lorsque l'utilisation du F22 comme liquide frigorifique sera interdite, il sera utile de pouvoir continuer à valoriser ce composé dans d'autres applications. La présente invention fournit un moyen particulièrement intéressant dans ce sens.

Il a en effet été trouvé que l'on pouvait transformer de façon sélective du chlorodifluorométhane (F22) en F134a et F32 en pyrolysant en continu le F22 à une température supérieure à 500°C en présence d'hydrogène, mais en l'absence de métaux dans la zone réactionnelle.

La quantité d'hydrogène mise en oeuvre est telle que le ratio molaire H₂/F₂₂ est compris entre 2 et 50, et plus particulièrement entre 5 et 15.

La pression de travail peut aller jusqu'à 100 bars, mais on opère généralement à une pression comprise entre 0,1 et 20 bars absolus, de préférence comprise entre environ 0,5 et 5 bars absolus et, plus particulièrement, à pression atmosphérique. La température de travail peut être comprise entre 500 et 1000°C, mais on opère préférentiellement entre 650 et 800°C.

Le temps de séjour peut être compris entre 0,1 et 100 secondes, mais on opère préférentiellement entre 1 et 20 secondes. En opérant avec de courts temps de séjour, on favorise la formation de F32, alors qu'avec de longs temps de séjour on augmente considérablement la production de F134a. Dans le cas où c'est ce dernier produit qui est intéressant, on peut évidemment recycler dans le réacteur le F32 co-produit pour le transformer en F134a.

Suivant les conditions opératoires, le F134a formé est accompagné d'une quantité variable de F134 (HF₂C-CHF₂) qui est facilement isomérisable en F134a.

Les exemples suivants illustrent de façon non limitative la présente invention.

### EXEMPLES 1 A 6

Tous les exemples sont réalisés dans un réacteur tubulaire en quartz de 47 cm de hauteur et de 2,3 cm de diamètre, placé dans un four électrique d'une puissance de 1,5 KW. La pression de travail est la pression atmosphérique et la température du four est mesurée à l'aide d'un thermocouple. Les réactifs sont introduits simultanément en continu par l'intermédiaire de rotamètres étalonnés permettant de contrôler les débits et donc les rapports molaires. Le flux des réactifs dans le réacteur peut être dilué par un débit de gaz inerte tel que l'hélium ou l'azote.

Le totalité du flux gazeux sortant du réacteur est acide et est envoyée dans un réacteur en verre contenant de la soude aqueuse pour éliminer l'acide chlorhydrique co-produit. Le flux gazeux sortant est ensuite séché sur tamis moléculaire, puis condensé à basse température (-78°C) dans un récipient en acier inoxydable muni de vannes permettant de stocker des produits gazeux à température ordinaire.

Les analyses des mélanges gazeux obtenus sont faites par chromatographie en phase gazeuse couplée à la spectrographie de masse de manière à identifier de façon certaine les produits de la réaction.

Le tableau suivant résume les conditions opératoires et les résultats obtenus.

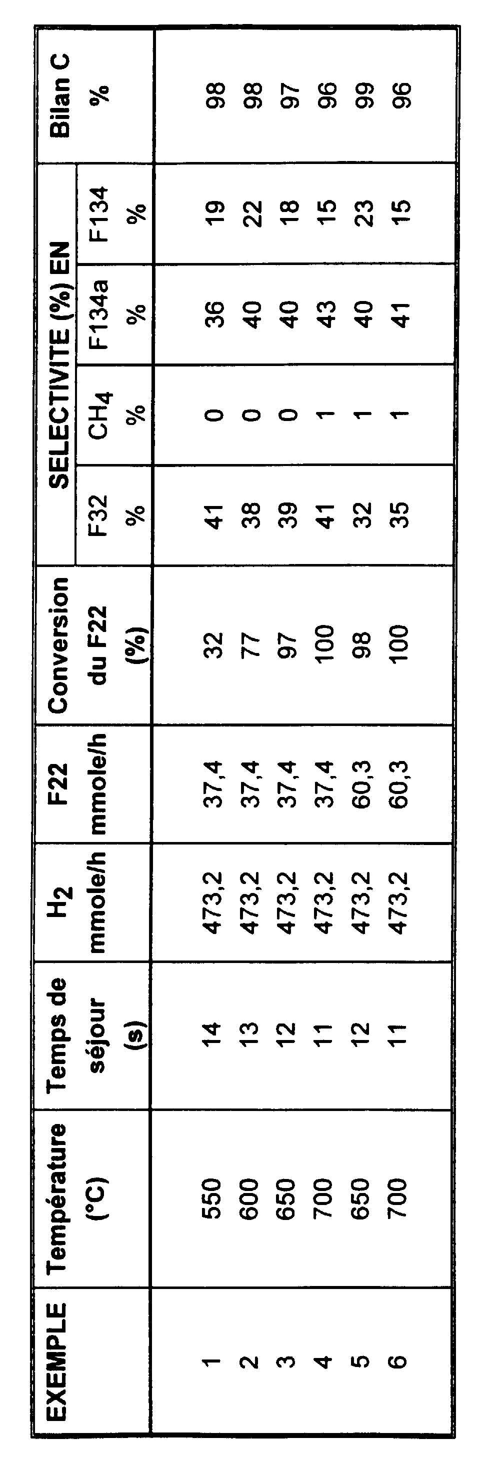

### EXEMPLE 7

On a opéré comme aux exemples précédents dans un réacteur en quartz de 47 cm de hauteur et 1,5 cm de diamètre, avec des débits d'hydrogène et de F22 de 218,8 mmole/h et 21 mmole/h respectivement. En travaillant à la pression atmosphérique et avec un temps de séjour de 12,4 secondes dans la zone isotherme (650°C), on a obtenu un taux de conversion du F22 de 93 % avec des sélectivités en F32, F134a et F134 de 26 %, 28 % et 18 % respectivement.

### EXEMPLE 8

On a opéré comme précédemment dans un réacteur en quartz de 47 cm de hauteur et 2,1 cm de diamètre, dans les conditions suivantes :
- température : 650°C
- temps de séjour dans la zone isotherme : 10 secondes
- pression : 0,48 bar absolu
- débit d'hydrogène : 218,8 mmole/h
- débit de F22 : 21 mmole/h

Le taux de conversion du F22 a été de 93 % avec des sélectivités en F32, F134a et F134 de 30 %, 30 % et 12 % respectivement.

### EXEMPLE 9

On a opéré dans le même réacteur qu'à l'exemple 7 dans les conditions suivantes :
- débit d'hydrogène : 513,4 mmole/h
- débit de F22 : 17,4 mmole/h
- température : 650°C
- pression : atmosphérique
- temps de séjour dans la zone isotherme : 2,8 secondes

Le taux de conversion du F22 a été de 92 % avec des sélectivités en F32, F134a et F134 de 34 %, 33 % et 18 % respectivement.

## Revendications

1. Procédé de coproduction de difluorométhane et de 1,1,1,2-tétrafluoroéthane caractérisé en ce qu'il consiste à pyrolyser du chlorodifluorométhane en présence d'hydrogène à une température supérieure à 500°C, en l'absence de tout catalyseur ou surface métallique.

2. Procédé selon la revendication 1 dans lequel on opère entre 500 et 1000°C, de préférence entre 650 et 800°C.

3. Procédé selon la revendication 1 ou 2 dans lequel le rapport molaire d'hydrogène au chlorodifluorométhane est compris entre 2 et 50, de préférence entre 5 et 15.

4. Procédé selon l'une des revendications 1 à 3 dans lequel le temps de séjour est compris entre 0,1 et 100 secondes, de préférence entre 1 et 20 secondes.

5. Procédé selon l'une des revendications 1 à 4 dans lequel on opère sous une pression comprise entre environ 0,1 et 20 bars absolus.

6. Procédé selon la revendication 5 dans lequel on opère sous une pression comprise entre environ 0,5 et 5 bars absolus, de préférence à la pression atmosphérique.

7. Procédé selon la revendication 6 dans lequel on opère dans un réacteur en quartz.

## Claims

1. Process for the co-production of difluoromethane and 1,1,1,2-tetrafluoroethane, characterized in that it consists in pyrolysing chlorodifluoromethane in the presence of hydrogen at a temperature of above 500°C, in the absence of any catalyst or metal surface.

2. Process according to Claim 1, which is carried out at between 500 and 1000°C, preferably between 650 and 800°C.

3. Process according to Claim 1 or 2, in which the molar ratio of hydrogen to chlorodifluoromethane is between 2 and 50, preferably between 5 and 15.

4. Process according to one of Claims 1 to 3, in which the residence time is between 0.1 and 100 seconds, preferably between 1 and 20 seconds.

5. Process according to one of Claims 1 to 4, which is carried out at a pressure of approximately between 0.1 and 20 bar absolute.

6. Process according to Claim 5, which is carried out at a pressure of approximately between 0,5 and 5 bar absolute, preferably at atmospheric pressure.

7. Process according to Claim 6, which is carried out in a quartz reactor.

## Patentansprüche

1. Verfahren zur gleichzeitigen Herstellung von Difluormethan und 1,1,1,2-Tetrafluorethan, dadurch gekennzeichnet, daß Chlordifluormethan in Gegenwart von Wasserstoff bei einer Temperatur über 500 °C in Abwesenheit von Katalysatoren oder Metalloberflächen pyrolisiert wird.

2. Verfahren nach Anspruch 1, bei dem man zwischen 500 und 1.000°C, vorzugsweise zwischen 650 und 800 °C arbeitet.

3. Verfahren nach Anspruch 1 oder 2, bei dem das Molverhältnis von Wasserstoff zu Chlordifluormethan zwischen 2 und 50, vorzugsweise zwischen 5 und 15, liegt.

4. Verfahren nach einem der Ansprüche 1 bis 3, bei dem die Verweilzeit zwischen 0,1 und 100 Sekunden, vorzugsweise zwischen 1 und 20 Sekunden, liegt.

5. Verfahren nach einem der Ansprüche 1 bis 4, bei dem man unter einem Druck zwischen etwa 0,1 und 20 bar absolut arbeitet.

6. Verfahren nach Anspruch 5, bei dem man bei einem Druck zwischen etwa 0,5 und 5 bar absolut, vorzugsweise bei Atmosphärendruck, arbeitet.

7. Verfahren nach Anspruch 6, bei dem man in einem Quarzreaktor arbeitet.
